(19) Europäisches Patentamt
European Patent Office
Office européen des brevets

(11) **EP 2 854 947 B1**

(12) **FASCICULE DE BREVET EUROPEEN**

(45) Date de publication et mention
de la délivrance du brevet:
**29.12.2021 Bulletin 2021/52**

(21) Numéro de dépôt: **13739745.1**

(22) Date de dépôt: **21.05.2013**

(51) Int Cl.:
*A61P 11/00* (2006.01)     *A61K 31/4355* (2006.01)
*A61K 31/4741* (2006.01)

(86) Numéro de dépôt international:
**PCT/FR2013/051101**

(87) Numéro de publication internationale:
**WO 2013/175116 (28.11.2013 Gazette 2013/48)**

(54) **UTILISATION DE LA TRITOQUALINE POUR LE TRAITEMENT DE LA MUCOVISCIDOSE**

VERWENDUNG EINES H4-AGONISTENMOLEKÜLS ZUR BEHANDLUNG VON ZYSTISCHER FIBROSE

USE OF AN H4 AGONIST MOLECULE FOR TREATING CYSTIC FIBROSIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.05.2012 FR 1201482**

(43) Date de publication de la demande:
**08.04.2015 Bulletin 2015/15**

(73) Titulaire: **Terrasse, Gaëtan**
**71300 Montceau-les-Mines (FR)**

(72) Inventeur: **Terrasse, Gaëtan**
**71300 Montceau-les-Mines (FR)**

(74) Mandataire: **Oudin, Stéphane et al**
**JurisPatent - Cabinet Guiu**
**10, rue Paul Thénard**
**21000 Dijon (FR)**

(56) Documents cités:
WO-A1-2013/164204     WO-A2-2007/072163
WO-A2-2008/031556     US-A1- 2010 144 718

• ZHANG ET AL: "The histamine H4 receptor: A novel modulator of inflammatory and immune disorders", PHARMACOLOGY AND THERAPEUTICS, ELSEVIER, GB, vol. 113, no. 3, 13 mars 2007 (2007-03-13) , pages 594-606, XP005918824, ISSN: 0163-7258, DOI: 10.1016/J.PHARMTHERA.2006.11.008
• S. P. Collingwood ET AL: "RESPIRATORY DRUG DISCOVERY, CURRENT DEVELOPMENTS AND FUTURE CHALLENGES", Drugs of the Furture, 1 août 2012 (2012-08-01), pages 619-625, XP055061153, DOI: 10.1358/dof.2012.37.8.1854604 Extrait de l'Internet: URL:http://journals.prous.com/journals/servlet/xmlxsl/dof/20123708/pdf/df370619.pdf?p_JournalId=2&p_refId=1854604&p_IsPs=N [extrait le 2013-04-25]

**Description**

La présente invention se rapporte à l'utilisation de substances chimiques les énantiomères lévogyres et dextrogyres de la (AMINO-7 TRIETHOXY-4,5,6 OXO-1 DIHYDRO-1,3 ISOBENZOFURANNYL-3)-1METHOXY-8 METHYL-2METHYLENEDIOXY-6,7TETRAHYDRO-,2,3,4ISOQUINOLEINE ou tritoqualine pour traiter les déficits respiratoires et les infections de la mucoviscidose.

La mucoviscidose (pour « maladie des mucus visqueux » en français) ou fibrose kystique (en anglais : Cystic fibrosis, sous-entendu « du pancréas ») est une maladie génétique, affectant les épithéliums glandulaires de nombreux organes.

C'est la maladie génétique létale à transmission autosomique récessive la plus fréquente dans les populations de type europoïde, alors qu'elle est très rare dans les populations africaines et asiatiques.

Elle est liée à des mutations du gène CFTR sur le chromosome 7, entraînant une altération de la protéine CFTR (sigle pour Cystic fibrosis transmembrane conductance regulator). Cette protéine est un canal ionique perméable au chlore, au thiocyanate dont la fonction est de réguler le transport du chlore à travers les membranes cellulaires.

Son dysfonctionnement provoque une augmentation de la viscosité du mucus et son accumulation dans les voies respiratoires et digestives. La maladie touche de nombreux organes mais les atteintes respiratoires sont prédominantes et représentent l'essentiel de la morbidité.

L'atteinte respiratoire se traduit par une baisse progressive du VEMS (Volume expiratoire moyen par seconde) et qui se transforme en une insuffisance respiratoire chronique.

Le poumon est en outre la cible d'infections chronique, du fait de la modification du mucus des cellules caliciforme du poumon.

Ce sont les infections et l'encombrement bronchique qui aboutit in fine a la détresse respiratoire, souvent à l'origine du décès.

En France, un nouveau-né sur 4200 est touché par la mucoviscidose. En effet, cette maladie génétique est assez fréquente. Ainsi 2 millions de personnes sont porteuses du gène et peuvent le transmettre à leur enfant. Environ 200 enfants naissent chaque année en France avec la mucoviscidose.

Il n'y a pas de traitement curatif mais les progrès de la prise en charge ont permis d'améliorer la qualité et l'espérance de vie des patients ; ainsi en France, l'espérance de vie à la naissance est passée de 7 ans en 1965 à 47 ans en 2005.

Il y a environ 70 000 patients en Europe et USA et c'est sur ces territoires que la pathologie est la plus importante. En Asie la prévalence de la maladie est très faible voir inexistante, comme au Japon. La prévalence de la maladie, met en évidence des variations importante de celle-ci (de 1 à 10). De 1 pour 2500 pour les européens à 1 pour 32 000 pour les asiatiques.

Le diagnostic biologique repose sur le test de la sueur confirmé par une identification des mutations génétiques. Le dépistage néonatal, généralisé en France depuis 2002 permet un diagnostic et une prise en charge précoce.

La mucoviscidose, décrite scientifiquement comme maladie en 1936, était en fait déjà connue depuis longtemps. Au Moyen Âge on connaissait le sort funeste du nouveau-né dont la mère remarquait le « baiser salé », c'est-à-dire le goût salé laissé par un baiser sur le front de l'enfant.

Au début du XXᵉ siècle apparaissent les premières observations associant maladie pulmonaire, diarrhée et anomalie pancréatique avec plusieurs cas dans la même famille. En 1912, Garrod décrit des familles dont les enfants présentent une diarrhée graisseuse et meurent d'infection pulmonaire.

C'est en 1936, dans une thèse écrite en allemand et présidée par le pédiatre suisse Guido Fanconi, que la maladie est décrite pour la première fois chez des enfants supposés atteints de maladie cœliaque, sous le nom de « fibrose kystique du pancréas et bronchectasie ».

La mucoviscidose ne fut considérée comme une entité pathologique distincte qu'en 1938 par la pédiatre américaine Dorothy Hansine Andersen, médecin au Babies' Hospital de New York, qui publia un article intitulé « fibrose kystique du pancréas et ses relations avec la maladie cœliaque »

En 1989, le gène impliqué dans la mucoviscidose est isolé par les équipes de Lap-Chi Tsui, Collins et Riordan. L'anomalie génétique à l'origine de la maladie est enfin découverte, il s'agit d'une mutation d'un gène localisé en 7q31 et contenant 27 exons, nommé *Cystic fibrosis* **(CF)** codant une protéine transmembranaire appelée *Cystic fibrosis transmenbrane conductance regulator* (**CFTR**) composée de 1480 acides aminés. Ce n'est qu'un peu plus tard qu'on apporta les preuves que CFTR était bien un canal du chlore.

La découverte de l'anomalie génétique permit par la suite d'ajouter le génotypage au protocole diagnostique, et d'envisager la thérapie génique.

Dans les années 1940, la maladie était considérée comme étant avant tout un problème nutritionnel avec une déficience en vitamine A. La prise en charge se résumait essentiellement à un régime riche en protéines, des injections intramusculaires de vitamine A à hautes doses, des extraits pancréatiques et des inhalations de pénicilline.

En 1945, Dorothy Andersen conseille « un régime pauvre en graisse, riche en protéines, avec une proportion libre de légumes, fruits et sucres et une restriction modérée en féculents.

La première drogue antibactérienne, un sulfamidé commercialisé sous le nom de Prontosil, fut disponible en 1934 et la pénicilline sous forme injectable en 1944. D'autres antibiotiques suivirent et eurent un rôle essentiel dans le traitement des patients.

A la fin des années cinquante, le principal germe pathogène était *Staphylococcus aureus* et de nombreuses souches étaient encore sensibles à la pénicilline. Lors de cette décennie, d'autres antibiotiques apparaissent. Dès 1951, alors que Staphylococcus aureus est la bactérie habituellement retrouvée, on note l'augmentation de la fréquence de Pseudomonas aeruginosa, attribuée aux traitements antibiotiques prolongés, néanmoins le bénéfice de traitements antibiotiques agressifs devient progressivement évident.

De nombreuses classes thérapeutiques sont utilisées actuellement dans la mucoviscidose mais les résultats ne sont pas toujours à la hauteur des espérances.

La thérapie génique : La mucoviscidose étant une maladie mono génétique, c'est-à-dire ne concernant qu'un seul gène, c'est naturellement que de grands espoirs de guérison sont nés avec l'apparition du concept de la thérapie génique.

Des analyses de la quantité d'ARN messager (ARNm) dans les cellules saines ont révélé un nombre extrêmement faible d'ARNm codant CFTR, entre deux et trois copies par cellule. En théorie, même avec un taux de transfert très faible, il serait possible de restaurer une fonction de sécrétion normale dans les cellules pulmonaires en apportant une ou deux copies du gène sain intégré dans un vecteur.

La fonction restaurée, le mucus devrait se fluidifier et permettre une clairance mucociliaire satisfaisante. En effet, les infections des voies pulmonaires par des pathogènes et l'inflammation qui s'ensuit est l'une des causes de la perte de la fonction respiratoire.

Mais malgré l'apparition de thérapie génique, les résultats n'ont donné jusqu'à ce jour aucun résultat probant.

La société copernic Therapeutics, a utilisé de l'ADN compacté (non viral) pour l'introduire par voie inhalée. Un essai de phase I a démontré des changements au niveau de la muqueuse nasale mais n'a pas démontré une meilleure expression du gène introduit par voir inhalée.

La modulation de l'expression du CFTR : De nombreuses sociétés ont des molécules en développement et en particulier la société VERTEX.

Cette dernière à lancé récemment en 2012 une molécule appelée Kalydeco qui améliore la fonction respiratoire, mais de manière peu importante (10% d'amélioration du VEMS).

Deux autres produits sont également en cours de développement l'Ataluren et le VX 809 dont les résultats cliniques sont également de faible amplitude sur la fonction respiratoire.

Les produits anti infectieux : Ce sont les infections et surinfections bronchiques aiguës ou chroniques qui vont détériorer progressivement la fonction respiratoire. Les germes principaux sont le *Staphylococcus*

*aureus,* le *Pseudomonas aeruginosa* et le *Burkholderia cepacia.* Ces germes sont la plupart résistant aux antibiotiques telles les pénicillines.

On utilise principalement les antibiotiques a base de Colimycine et tobramycine. Les produits sont des formes inhalés soit sous forme sèche (Tobi podhaler, colobreathe) ou humide (Tadim, Tobi, cayston, Colimycine).

D'autres produits sont également en cours d'évaluations : L'Azithromycine de chez Pfizer - Chez les patients porteurs chroniques du Pseudomonas aeruginosa, cet antibiotique utilisé par voie inhalée permet un net gain de poids et la diminution du nombre d'hospitalisation.

L'Aztreonam de Gilead Sciences est un antibiotique sous forme inhalée qui a reçu l'approbation de FDA le 22 Février 2010 et est disponible pour les patients américains.

Il existe également plusieurs autres produits en développement comme l'Arikace une formulation liposomale de l'Amikacine, mais également le KB001 de KaloBios Pharmaceuticals qui a lancé un essai de phase 1 pour tester la sécurité de leur approche du traitement des infections à Pseudomonas aeruginosa par anticorps.

D'autres antibiotiques sont également en cours de développement pour traiter les infections de la mucoviscidose comme DMP-376 est une nouvelle formulation de la lévofloxacine ainsi que le GS 9310/11de Gilead Sciences une combinaison de fosfomycine et la tobramycine par voie inhalée.

La voie des produits anti-inflammatoires a également été explorée comme avec l'Ibuprofène, le N acétylcystéine, le Docosahexaenoic acide (DHA), le KB001 anticorps monoclonal humanisé, le GSK SB 656933, ainsi que le Sildénafil.

Dans cette classe seul l'ibuprofène à fait la preuve de son efficacité a un faible cout mais les résultats obtenus sont de faible ampleur.

Les produits utilisés dans la bronchite chronique et l'asthme n'ont pas donné de résultats.

Il y a pourtant un fort potentiel dans cette classe thérapeutique, car la mucoviscidose est avant tout une maladie inflammatoire du poumon et du tube digestif.

Les modificateurs de mucus sont également une classe thérapeutique qui se développe. Dans la mucoviscidose, des changements dans le transport du sel dans les cellules rendent le mucus très épais et collant. Cette approche cible les protéines autres que la protéine CFTR à améliorer la circulation de sel dans et hors des cellules, ce qui permet au mucus d'être plus hydratée et, par conséquent, plus facilement éliminé.

Plusieurs produits sont utilisés ou en cours de développement comme La solution saline hypertonique, Le Denufosol de Inspire Pharmaceuticals qui permet de corriger le défaut de transport ionique dans les cellules pulmonaires. Ce produit augmente le VEMS par rapport au placebo. On trouve également le Bronchitol (Mannitol) de Pharmaxis qui théoriquement devrait aider à réhydrater les sécrétions pulmonaires.

Dans cette classe on trouve 3 autres produits en développement, le SPI-8811 de Sucampo Pharmaceuticals et également le 1901 de la société Lantibio, ainsi que le Gilead GS9411 qui agit en bloquant l'absorption de sodium.

Dans cette classe de produit, la solution hyper saline est déjà sur le marché avec plus de 10 000 patients traités. Le cout du traitement est faible et entre directement en concurrence avec les nouveaux produits en développement de chez Pharmaxis (Bronchitol) et de GSK (GSK9411) dont les résultats sur le VEMS ne sont pas supérieurs à la solution hyper saline.

L'altération du mucus est aussi actuellement traitée par le Pulmozyme de chez Genentech sur plus de 18 000 patients dans le monde.

Malgré de nombreuses classes thérapeutiques utilisées, si la survie des patients atteints par la mucoviscidose a augmenté, le résultat des traitements est encore très insuffisant.

La tritoqualine est une substance chimique connue depuis de très nombreuses années, et utilisée comme antihistaminique. Sa fabrication est décrite dans le brevet français FR 1.295.309.

La tritoqualine est la 7-Amino-4,5,6-triethoxy-3-(5,6,7,8-tetrahydro-4-methoxy-6-methyl-1,3-dioxolo[4,5-g]isoquinolin-5-yl) phthalide. Dans sa forme pharmaceutique commercialisée elle se présente sous la forme d'un mélange d'énantiomères.

La tritoqualine est connue pour son activité antiallergique par son action inhibitrice sur l'histidine décarboxylase. Cette activité est cependant très faible et n'explique pas les nombreuses propriétés qu'elle présente sur des symptômes cliniques variés, rhinite, urticaire, eczéma, mastocytose.

L'inventeur avec d'autres inventeurs a démontré que la tritoqualine avait une action très importante sur un nouveau récepteur, le récepteur H4 à l'histamine.

Cette activité de la tritoqualine sur le récepteur H4 a été démontrée dans un brevet US (US2010144718A1) « TREATMENT OF DISEASES MODULATED BY A H4 RECEPTOR AGONIST » Gaëtan terrasse et coll. récemment accepté.

Ce brevet ne décrit cependant pas l'activité de la tritoqualine sur la mucoviscidose.

Un autre brevet sur les agonistes H4 décrit l'utilisation de ces produits dans la protection des précurseurs hématopoïétiques dans le cadre de chimiothérapie. WO2008006974A2 "use of histamine h4 receptor ligands to protect haematopoietic progenitors against the haematological toxicity of chemotherapeutic agents" Michel DY et coll.

Ce dernier brevet et Aucuns autres brevets ou documents scientifiques ne mettent en évidence l'action des agonistes H4 à l'histamine sur la mucoviscidose.

La tritoqualine commerciale se présente sous la forme d'une poudre blanche, très sensible à la lumière qui la dégrade en Cotarnine et acide phtalique.

La tritoqualine commerciale (appelée Hypostamine) est sous la forme de comprimé avec une concentration de 100 mg par comprimé.

La tritoqualine présente 2 carbones asymétriques, mais la forme commerciale est un mélange de 2 énantiomères.

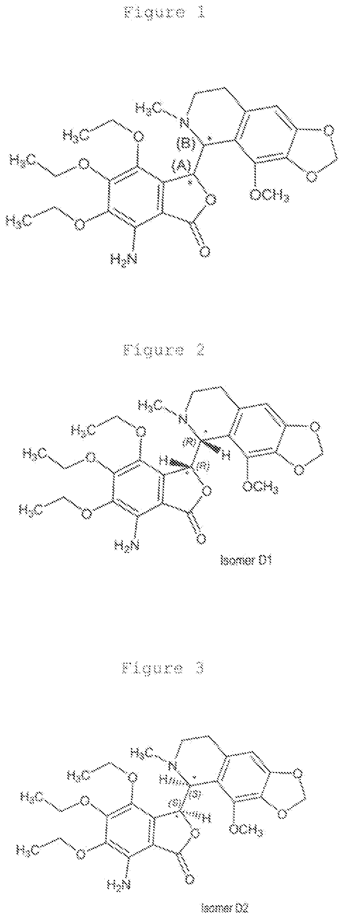

La Figure 1 illustre la présence des carbones asymétriques, qui sont notés A et B. La Figure 2 illustre la forme de l'isomère D1, et la Figure 3 illustre La forme de l'isomère D2.

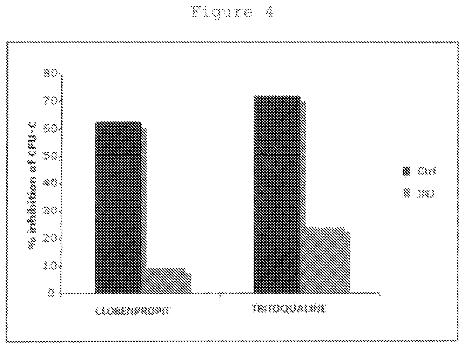

La figure 4 compare la tritoqualine et le Clobenpropit, agoniste H4 de référence. Cette figure met en évidence que la tritoqualine est capable d'inhiber la prolifération des CFU riche en récepteur H4. Quand on utilise un anti H4, l'activité de la tritoqualine est fortement diminuée comme celle du Clobenpropit. Ceci démontre bien que la tritoqualine est une molécule agoniste H4.

Il a été mis en évidence les propriétés étonnantes de la tritoqualine commerciale dans la mucoviscidose.

La tritoqualine est utilisée à la dose de 100 mg à 800 mg et préférentiellement à une dose de 200 à 400 mg dans les exemples suivants.

## Exemple 1

Il s'agit d'un enfant de 10 ans atteint de mucoviscidose et qui présente une atteinte respiratoire importante. Lors de la consultation initiale, un traitement à base de tritoqualine est mis en route à la dose quotidienne de 200 mg.

Avant la thérapie avec la tritoqualine, le VEMS (Volume expiratoire Maximal en 1 seconde) de ce jeune patient est de 0,39 L /s soit 55,71% de la valeur normale attendue (fondée sur l'âge, le sexe, et la taille). La mesure des débits est effectuée avec un appareil de pléthysmographie JAEGER® MasterScreen® Body.

Ce niveau de débit ventilatoire signe une atteinte respiratoire sévère. Ce patient a fait en outre 15 infections pulmonaires par an avant le traitement. Après 6 mois de traitement avec la tritoqualine à la dose de 200 mg/jour, les débits ventilatoires sont de nouveau mesurés. A cette deuxième visite le paramètre de VEMS est mesuré à 0.50 L/s. Il s'agit d'une augmentation de 28,2% par rapport aux débits initiaux mesurés lors de la visite initiale. Lors de la troisième visite à 12 mois de la première visite, les débits sont de nouveau mesurés. Le VEMS est alors de 0.62 L/s soit une amélioration de 58.7% par rapport aux débits initiaux.

Sur 12 mois le nombre de surinfections est passé de 15 à seulement 4 infections. Ainsi de manière surprenante la tritoqualine a augmenté de plus de 50% les débits ventilatoires de ce jeune patient et divisé par 3 le nombre d'infections.

## Exemple 2

Un adulte de 18 ans atteint de mucoviscidose est vu en consultation une première fois durant laquelle un traitement de tritoqualine est mis en route à la dose de 400 mg par jour. Avant la thérapie avec tritoqualine, le VEMS du patient est mesuré à 0,45 L/s soit à seulement 69,23% de la valeur normale attendue (fondée sur l'âge, le sexe, et la taille).

Ce patient a fait 8 infections pulmonaires par an avant le traitement. A 6 mois de traitement a la dose quotidienne de 400 mg de tritoqualine, le paramètre de VEMS a été mesurée de nouveau lors d'une deuxième consultation. Le paramètre VEMS mesuré lors de cette consultation passe de 0.45 L/s à 0.50 L/s soit une amélioration de 11,11% des débits ventilatoires.

Une troisième consultation après 12 mois de traitement montre que le paramètre VEMS, passe de 0.50 L/s à 0.58 L/s. Cette augmentation se traduit par rapport à la visite initiale par une augmentation de 28.89% du VEMS (100 * (0.58-0.45) / 0,45 par rapport au départ).

Pendant le traitement de 12 mois avec tritoqualine (400 mg par jour), ce patient a bénéficié d'une réduction significative des infections pulmonaires, de 8 au départ à seulement 3 des infections après 12 mois de traitement.

Cette diminution des infections et l'augmentation des débits ventilatoire et tout a fait surprenante, d'autant que les traitements usuels de ce patient n'avaient aucun effet sur sa fonction respiratoire.

**Exemple 3**

Un enfant de 12 ans atteint de mucoviscidose est traité lors de sa première consultation à la dose de 300 mg de tritoqualine.

Avant la thérapie avec tritoqualine, le VEMS du patient est mesuré a 0,70 L/s, soit 63,64% de la valeur normale attendue (fondée sur l'âge, le sexe, et la taille). Ce patient a fait 12 infections pulmonaires par an avant le traitement. En 6 mois de traitement avec la dose quotidienne de 300 mg de tritoqualine, le paramètre VEMS a été mesurée lors de sa deuxième consultation a 1.0 L/s. Ceci représente une amélioration de 42,86% par rapport à la visite initiale. (100 * (1,00 à 0.0,70) / 0,70). A 12 mois de traitement le paramètre VEMS mesuré lors de la troisème consultation est de 1,20 L/s soit (71,43% d'amélioration par rapport à la visite initiale.

$$(100 * (1.20-0.70) / 0,70)$$

Pendant le traitement de 12 mois avec tritoqualine a raison de 300 mg par jour, ce patient a eut une réduction significative des infections pulmonaires. Ces dernières sont passées de 12 à 3 infections sur une seule année.

On remarque ainsi que la tritoqualine permet de diminuer le nombre des surinfections chez les patients de manière surprenante et augmente de manière parallèle les débits ventilatoires. La tritoqualine a de plus un effet sur les débits ventilatoire particulièrement important , bien supérieur à tous les traitement existant à ce jour. Nous pouvons donc affirmer que la tritoqualine sous sa forme classique de mélange d'énantiomères à la dose de 100 à 400 mg permet de traiter les dégradations respiratoires de la mucoviscidose. Le traitement permet en outre une réduction significative des surinfections bronchiques ainsi que des hospitalisations.

La tritoqualine pourrait être utilisée sous différentes formes, en dehors de sa forme comprimé sans modifier son efficacité, par exemple sous forme de gélules, sirop, gel oral ou comprimés à délitement programmé.

## Revendications

**1.** Substance constituée d'un isomère ou une mixture d'isomères de la 7-amino-4,5,6-trietoxy-3-(5, 6, 7, 8-tétrahydro-4methoxy-6-methyl-1, 3-dioxolo (4, 5, g) isoquinolin-5-yl) phtalide, ou tritoqualine pour son utilisation dans le traitement de la mucoviscidose à une dose de 100 à 800 mg par jour **caractérisée en ce qu'**elle est conditionnée sous forme de comprimés, gélules, capsules, sirop ou gel oral.

## Patentansprüche

**1.** Substanz, die aus einem Isomer oder einer Mischung von Isomeren von 7-Amino-4,5,6-triethoxy-3-(5,6,7,8-tetrahydro-4methoxy-6-methyl-1,3-dioxolo(4,5,g)isochinolin-5-yl)phthalid oder Tritoqualin besteht, zu deren Verwendung bei der Behandlung von Mukoviszidose in einer Dosis von 100 bis 800 mg täglich, **dadurch gekennzeichnet, dass** sie in der Form von Tabletten, Hartkapseln, Kapseln, Sirup oder oralem Gel dargereicht wird.

## Claims

**1.** Substance consisting of an isomer or a mixture of isomers of 7-amino-4,5,6-triethoxy-3-(5,6,7,8-tetrahydro-4-methoxy-6-methyl-1,3-dioxolo(4,5,g)isoquinolin-5-yl)phthalide, or tritoqualine for use in the treatment of cystic fibrosis in a dose of 100 to 800 mg per day, **characterised in that** it is presented in the form of tablets, caps, capsules, syrup or oral gel.

Figure 1

Figure 2

Isomer D1

Figure 3

Isomer D2

Figure 4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1295309
- US 2010144718 A1

- WO 2008006974 A2